# EUROPÄISCHE PATENTSCHRIFT

(11) **EP 2 389 206 B1**
(45) Veröffentlichungstag und Bekanntmachung des Hinweises auf die Patenterteilung: **06.07.2016**
(21) Anmeldenummer: 10705777.0
(22) Anmeldetag: 20.01.2010
(51) Int. Cl.: A61M 1/36

(54) **SORPTIONSMITTEL ZUM ENTFERNEN PROTEINGEBUNDENER SUBSTANZEN**
SORBENT FOR REMOVING PROTEIN-BOUND SUBSTANCES
SORBANT POUR ÉLIMINER DES SUBSTANCES LIÉES À DES PROTÉINES

(30) Priorität: 22.01.2009 AT 1122009
(43) Veröffentlichungstag der Anmeldung: 30.11.2011
(73) Patentinhaber: Fresenius Medical Care Deutschland GmbH, 61352 Bad Homburg (DE)
(72) Erfinder: FALKENHAGEN, Dieter, A-3500 Krems (AT); HARTMANN, Jens, A-3511 Furth (AT); WEBER, Viktoria, A-3500 Krems/Donau (AT)
(74) Vertreter: Patentanwaltskanzlei Matschnig & Forsthuber OG
(86) Internationale Anmeldenummer: PCT/AT2010/000016
(87) Internationale Veröffentlichungsnummer: WO 2010/083544

(56) Entgegenhaltungen:
- EP-A1- 0 909 565
- GB-A- 2 000 150
- US-A1- 2007 181 499

## Beschreibung

Die Erfindung betrifft ein Sorptionsmittel zum Entfernen proteingebundener Substanzen aus einer biologischen Flüssigkeit.

Der Leber als zentrales Stoffwechselorgan kommen wichtige Aufgaben im Glucose-, Eiweiß- und Fettstoffwechsel, in der Speicherung von Kohlenhydraten und Vitaminen, der Entgiftung und der Immunabwehr zu. Toxische Substanzen und Stoffwechselprodukte, die sich im Blut ansammeln, werden von einer funktionierenden Leber aus dem Blut entfernt Bei Patienten mit verminderter Leberfunktion oder einem akuten bzw. einem aufgrund einer chronischen Erkrankung hervorgerufenen Leberversagen muss das Blut von angereicherten, toxischen Substanzen befreit werden. Auch bei Personen mit Niereninsuffizienz kommt es zu einer Anreicherung von zu entfernenden Lebergiften im Blut. Ein Leberversagen erfordert eine sofortige klinische Behandlung, da die Anreicherung toxischer Substanzen in kurzer Zeit zu Multinrgankomplikationen bis hin zum Versagen verschiedener Organe wie Niere und Lunge führt. Eine Entgiftungsbehandlung erfolgt mit Hilfe eines künstlichen Leberunterstützungssystems ("künstliche Leber", Leberdialyse), mit welchem toxische Substanzen effektiv aus dem Blut entfernt werden können. Dadurch können Patienten mit lebensbedrohlichem Leberversagen bis zum Zeitpunkt der Transplantation stabilisiert und am Leben erhalten werden. Da die Leber eine hohe Selbstregenerationsfähigkeit aufweist, kann eine die Leber entlastende Behandlung sogar eine Wiederaufnahme der Leberfunktion des Patienten bewirken. Ausschlaggebend für den Behandlungserfolg ist die Effektivität des Leberunterstützungssystems.

Die toxischen Substanzen im Blut von Leberkranken können in wasserlösliche und wasserunlösliche eingeteilt werden. Beispiele toxischer Substanzen, die mittels eines Leberunterstützungssystems aus dem Blut entfernt werden, sind unkonjugiertes Bilirubin, Gallensäuren, vorrangig aromatische Aminosäuren, phenolische Verbindungen und Ammoniak. Wasserunlösliche Lebergifte liegen zum Transport im Blut proteingebunden - zumeist an das Bluteiweiß Albumin - vor. Bilirubin wird beim Abbau des Hämoglobins und anderer Hämproteine (Myoglobin, Cytochrome) gebildet. Da es nicht wasserlöslich ist, kann es nicht über die Niere ausgeschieden werden. Zum Transport im Blut ist Bilirubin an Albumin gebunden (unkonjugiertes oder indirektes Bilirubin). In der Leber wird Bilirubin an Glucuronsäure gekoppelt (konjugiertes oder direktes Bilirubin). Konjugiertes Bilirubin ist wasserlöslich und kann mit der Galle in den Darm bzw. in die Nieren ausgeschieden werden. Bei einem Versagen der Leberfunktion kann kein konjugiertes Bilirubin mehr gebildet und unkonjugiertes Bilirubin reichert sich im Blut an. Bei einem erhöhten Serumbilirubinspiegel kommt es dann zur Gelbsucht mit Gelbfärbung der Skleren, der Haut und, bei einem stark erhöhten Bilirubinspiegel, auch der Gewebe und der Organe. Bilirubin stellt daher einen wichtigen Marker für eine Störung bzw. einen Ausfall der Leberfunktion dar.

Extrakorporale Leberunterstützungssysteme sind aus dem Stand der Technik bekannt. Zum Entfernen proteingebundener Substanzen weisen extrakorporale Leberunterstützungssysteme üblicherweise eine Sorptionseinrichtung (Adsorber) auf, die im extrakorporalen Blutkreislauf bzw. im Plasmakreislauf angeordnet ist und mittels welcher die proteingebundenen Substanzen aus dem Blut bzw. dem Plasma eliminiert werden. Die Sorptionseinrichtung enthält ein Sorptionsmittel (Adsorbens). Dabei ist die Effizienz der Sorptionseinrichtung bzw. die Geschwindigkeit der Sorption (insbesondere Adsorption) durch das Sorptionsmittel elementar für den Behandlungserfolg bzw. für die überbrückende Stabilisierung eines Patienten, insbesondere eines Patienten mit Leberversagen.

Der Kontakt von Blut bzw. Plasma mit Fremdoberflächen führt zur Kontaktaktivierung und in weiterer Folge zur Gerinnung, zur Freisetzung von Kininen und zur Aktivierung des Komplementsystems. Diese Prozesse werden auch durch extrakorporale Blutreinigungsverfahren ausgelöst, da Plasma bzw. Blut im Rahmen dieser Verfahren mit Membranen und Adsorbern in Kontakt gebracht werden. Bei den meisten Patienten, die einer extrakorporalen Blutreinigung bedürfen, ist daher eine Hemmung der Blutgerinnung erforderlich. Standardmäßig erfolgt dies mittels Heparininfusion. Für Patienten, für die eine Heparingabe kontraindikativ ist, kann eine regionale Antikoagulation mit Citrat erfolgen.

Adsorber auf Basis eines Anionenaustauschers kommen sehr häufig in extrakorporalen Leberunterstützungssystemen zur klinischen Anwendung. Als bekannte Vorrichtungen, in welchen Anionenaustauscher zur klinischen Anwendung kommen, sind die Leberdialysevorrichtung MARS (Molecular Adsorbent Recirculation System) und das Prometheus-System zu nennen. Mittels eines Anionenaustauschers können proteingebundene, toxische Substanzen wie Bilirubin sehr effizient aus einer biologischen Flüssigkeit wie Blutplasma entfernt werden. Insbesondere das Prometheus-System zeichnet sich durch eine hohe Clearance proteingebundener und wasserlöslicher Lebergifte aus, wodurch die Überlebensrate von Leberpatienten stark gesteigert werden konnte. Anionenaustauscher haben jedoch den großen Nachteil, dass nicht nur die gewünschten proteingebundenen Substanzen, sondern auch physiologisch wichtige Proteine bzw. Faktoren des Blutgerinnungssytems (Antikoagulanzien sowie Prokoagulanzien) in sehr hohem Maße eliminiert werden. Hier sind insbesondere patienteneigene Gerinnungshemmer wie Protein C und Protein S zu nennen. Von besonderer Wichtigkeit ist das Protein C als gerinnungshemmender Faktor. Eine Verminderung der intrakorporalen Protein C-Konzentration führt zu schweren Gerinnungskomplikationen wie Venenthrombosen und Lungenembolien. Auch das per Infusion zusätzlich in den extrakorporalen Blutkreislauf zugeführte Antikoagulans Heparin wird durch den Anionenaustauscher eliminiert. Die Folge ist ein Ungleichgewicht des intrakorporalen Gerinnungssystems bzw. eine unerwünschte Antikoagulation im extrakorporalen bzw. intrakorporalen Blutkreislauf. Dies kann besonders für Hochrisikopatienten wie Patienten mit Leberversagen und Sepsis sehr problematisch sein. Über das große Risiko einer okklusiven Thrombosebildung im Rücklauf des extrakorporalen Blutreinigungssystems bzw. im Patienten nach Rückführung des gereinigten Blutes durch die unerwünschte Bindung von Proteinen des Blutgerinnungssystems am Anionenaustauscher wurde kürzlich berichtet [Meijers et al. 2007. Major Coagulation Disturbances During Fractionated Plasma Separation and Adsorption. Am J Transplant 7(9):2195-2199].

Aufgrund der oben beschriebenen Nachteile eines Anionenaustauschers wurde die Verwendung eines neutralen, hydrophoben Polymers vorgeschlagen. Das Polymer weist Poren auf, in welchen die Substanzen adsorbiert werden. Die gewählte Porengröße des Polymers ist dabei ein entscheidender Faktor. Es konnte in *vitro* gezeigt werden, dass nur Poren größer als 5-6 nm für stark an Proteine gebundene Substanzen wie Bilirubin zugänglich sind. Bei einer mittleren Porengröße von > 7,5 nm wurde eine gute Bilirubin-Adsorption festgestellt [Weber et al. 2008. Neutral styrene divinylbenzene copolymers for adsorption of toxins in liver failure. Biomacromolecules 9(4):1322-1328]. Die Adsorption proteingebundener Substanzen ist bei höheren Porengrößen aufgrund der besseren Porenzugänglichkeit besser. Allerdings sinkt die Kapazität und damit die Adsorptionsleistung des Adsorbers bei einer zu groß gewählten Porengröße wieder, da die innere Oberfläche des Polymers mit ansteigender Porengröße sinkt.

Für neutrale, hydrophobe Polymere konnte weiters festgestellt werden, dass - im Gegensatz zu den oben beschriebenen Anionenaustauschern - nur wenig Heparin gebunden wird. Darüber hinaus ist die Bildung von Thrombin-Antithrombin-Komplexen stark herabgesetzt Nachteilig ist jedoch, dass auch durch ein neutrales, hydrophobes Polymer mit einer mittleren Porengrößen > 7,5 nm eine ausgeprägte Adsorption des Protein C erfolgt. Es ist anzunehmen, dass auch andere wichtige Proteine ähnlichen Molekulargewichts wie beispielsweise Protein S adsorbiert werden. Bei kleineren, für stark proteingebundene Substanzen unzugänglichen Porengrößen (< 5-6 nm) wurde Protein C nicht adsorbiert.

Durch die Verwendung eines neutralen, hydrophoben Polymers anstelle eines Anionenaustauschers konnte somit ein zumindest teilweise selektives Sorptionsmittel bereitgestellt werden, da die Heparin-Adsorption stark gesenkt bzw. verhindert werden konnte. Die Adsorption von Protein C durch neutrale, hydrophobe Polymere ist jedoch immer noch so signifikant, dass dies zu der bereits oben beschriebenen Gerinnungsproblematik führen kann. Diese Gefahr besteht insbesondere bei Patienten, bei welchen eine Heparingabe kontraindikativ ist. Die Verwendung von Polymeren kleinerer Porengröße, bei welchen Protein C nicht adsorbiert wird, ist nicht zu befürworten, da die Entfernung proteingebundener Substanzen ineffizient ist. Die Adsorptionseffizienz für proteingebundene Substanzen gilt, wie bereits betont, als entscheidender Überlebensfaktor bei Leberversagen.

Ein System besonders hoher Leistung in Bezug auf die Entfernung proteingebundener Substanzen ist das Microspheres-based Detoxificaton System (MDS), welches aus der EP 0776223 B1 und der US 5855782 bekannt geworden ist. Über Microspheres-Adsorberpartikel (mittlere Partikelgröße 5 µm) hergestellt aus einem neutralen, hydrophoben Polymer (Styrol/Divinylbenzol-Copolymer, mittlerer Porendurchmesser: > 100 nm) wurde kürzlich berichtet [Falkenhagen D., Vienken J., The Prometheus-System. Technical Background and Clinical Experience. ASAIO 54th Annual Conference, 19-21 June 2008].

Neutralharze zum Entfernen proteingebundener Substanzen sind weiters in der WO 2005/082504 A2 offenbart. Die WO 2005/082504 A2 beschreibt eine Entgiftungsvorrichtung, welche Aktivkohle und zumindest ein nicht-ionisches Harz mit einer mittleren Porengröße von 30 nm (Polystyrendivinylbenzenharz - Amberchrom GC 300C) bzw. 45 nm (Harz auf Basis aliphatischer Ester - Amberlite XAD-7HP) aufweist. Die oben beschriebene Problematik neutraler, hydrophober Polymere trifft auch für diese Neutralharz-Adsorber zu.

Ein Sorptionsmittel sowie ein Verfahren zur Herstellung eines Sorptionsmittels der eingangs genannten Art wurde bereits Ende der 1970er Jahre offenbart [Ton et al. 1979. Adsorption of Human Serum Albumin to Amberlite XAD-7 Resin. J Biomed Mater Res 13:407-422], [Hughes et al. 1978. The use of an in vitro haemoperfusion circuit to evaluate the blood compatibility of albumin-coated Amberlite XAD-7 resin. Int J Artif Organs 1(3):129-34], [Ton H.Y. et al. 1979. Albumin-coated Amberlite XAD-7 resin for hemoperfusion in acute liver failure. Part I: adsorption studies. Artif Organs 3(1):20-22], [Hughes R. et al. 1979. Albumin-coated Amberlite XAD-7 resin for hemoperfusion in acute liver failure. Part II: in vivo evaluation. Artif Organs. 3(1):23-26], [Falkenhagen et al. 1981. Optimization of albumin coating for resins. Artif Organs 5 (Suppl):195-199]. Die adsorptive Beschichtung von neutralen, hydrophoben Harzen wie Amberlite XAD-7 (mittlere Porengröße: 9 nm) bzw. Y56 (mittlere Porengröße: 8 nm) mit einer Polypeptidschicht (Albumin) hatte das Ziel, die Bindung von Thrombozyten an den Adsorber und den damit verbundenen Thrombozytenverlust während einer Hämoperfusion zu verringern.

In einer weiteren, jüngeren Publikation, welche ein Sorptionsmittel der eingangs genannten Art beschreibt, wurde berichtet, dass Bilirubin an einem unbeschichteten Neutralharz (Polystyroldivinylbenzol, Porengröße 5-10 nm) besser adsorbiert wird als an einem mit einer Albuminschicht beschichteten Neutralharz [Annesini et al., 2005. Bilirubin removal from albumin-containing solution by adsorption on polymer resin. The International Journal of Artificial Organs. Vol 28, Nr. 7: 686-693]. Die am Neutralharz adsorbierte Albuminschicht wirkt sich folglich negativ auf die Bilirubin-Adsorption und somit auf die Adsorptionseffizienz aus.

US 2007/181499 A1 und GB 2 000 150 A offenbaren Adsorptionsmittel umfassend neutrale, hydrophobe Polymere, die mit Humanalbumin beschichtet sind.

Die EP 0 909 565 A1 beschreibt die Verwendung eines Styrol-Divinylbenzol-Copolymer-Herzes zur Adsorption von Bilirubin, Gallensäuren und deren Derivaten aus menschlichem Blut oder Plasma.

Es ist eine Aufgabe der Erfindung, ein verbessertes Sorptionsmittel bereitzustellen. Es soll ein Sorptionsmittel zum Entfernen von proteingebundenen Substanzen aus einer biologieschen Flüssigkeit mit signifikant verbesserten Selektivitätseigenschaften im Vergleich zu den aus dem Stand der Technik bekannten Sorptionsmitteln bereitgestellt werden. Das verbesserte Sorptionsmittel soll proteingebundene Substanzen im Blut von Leberpatienten hocheffizient eliminieren, während an der Blutgerinnung beteiligte Proteine, insbesondere wichtige physiologische gerinnungshemmende Faktoren wie das Protein C oder das Protein S in der biologischen Flüssigkeit verbleiben.

Diese Aufgabe wird durch ein Sorptionsmittel gemäß den Merkmalen des Kennzeichens des Anspruchs 1 gelöst.

Dank der Erfindung wird ein Sorptionsmittel mit deutlich verbesserten Selektivitätseigenschaften zur Verfügung gestellt, mit welchem einerseits ein hocheffizientes Entfernen proteingebundener Substanzen gewährleistet wird und andererseits die Gerinnungsproblematik, welche mit neutralen, hydrophoben Polymeren in Verbindung steht, umgangen werden kann. Mit dem erfindungsgemäßen Sorptionsmittel wird erstmals die Möglichkeit geschaffen, einerseits proteingebundene Substanzen effizient aus dem Blut von Patienten mit schweren Störungen der Leberfunktion bzw. Leberversagen zu entfernen und andererseits schwerwiegenden Gerinnungskomplikationen zu verringern und die Patientensicherheit dadurch zu verbessern.

Protein C, ein Vitamin K abhängiges Protein im Blutplasma, ist ein wichtiger Regulator des Blutgerinnungsablaufs und hat eine antikoagulatorische Wirkung. Eine unerwünschte Bindung des Protein C an unbeschichtete neutrale, hydrophobe Polymere, wie sie aus dem Stand der Technik bekannt sind, kann daher zu Gerinnungskomplikationen (Venenthrombosen, Lungenembolien) führen. Es ist mit großer Wahrscheinlichkeit anzunehmen, dass auch die Bindung von Protein S durch das erfindungsgemäße Sorptionsmittel deutlich gesenkt wird, da Protein S (62 000 Da) ein ähnliches relatives Molekulargewicht wie das Protein C (69 000 Da) aufweist. Dieselben Überlegungen gelten für Gerinnungsfaktoren ähnlichen Molekulargewichts (z.B. Faktor VII, Faktor IX und Faktor X).

Darüber hinaus wurde festgestellt, dass die Sorptionsleistung des erfindungsgemäßen Sorptionsmittels in Bezug auf die zu entfernenden proteingebundenen Substanzen (z.B. Bilirubin) im Vergleich zu einem unbeschichteten Polymer nicht bzw. nur unwesentlich beeinträchtigt wird. Das erfindungsgemäße Sorptionsmittel weist daher auch in diesem Punkt einen Vorteil zu dem oben beschriebenen, aus dem Stand der Technik bekannten Sorptionsmittel [siehe Annesini et al., 2005. Bilirubin removal from albumin-containing solution by adsorption on polymer resin. The International Journal of Artificial Organs. Vol 28, Nr. 7: 686-693] auf.

Im Folgenden sollen zum besseren Verständnis einige der verwendeten Begriffe näher erläutert werden:

Unter dem Begriff "Sorptionsmittel" im Rahmen dieser Offenbarung ist ein Mittel zum Durchführen einer Sorption, vorzugsweise einer Adsorption, zu verstehen, d.h. Moleküle, die sich in einer biologischen Flüssigkeit befinden, werden durch die Oberflächenkräfte des Sorptionsmittels festgehalten. In der Beschreibung werden daher anstelle des Begriffs "Sorptionsmittel" auch die Begriffe "Adsorbtionsmittel" bzw. "Adsorbens" bzw. "Adsorber" verwendet.

Der im Rahmen der Erfindung verwendete Ausdruck "biologische Flüssigkeit" kann sich auf zellfreie Flüssigkeiten, insbesondere Blutplasma, oder auf Zellen enthaltende Flüssigkeiten, insbesondere Blut, beziehen. Da es im Zuge einer künstlichen Leberunterstützung auch notwendig ist, andere Flüssigkeiten wie beispielsweise Gerinnungshemmer enthaltende Lösungen (Heparin-Lösung, Citrat-Lösung) oder Substitutionslösungen (Elektrolyte, Flüssigkeiten zum Ausgleich des Flüssigkeitsverlust) in den extrakorporalen Blutkreislauf bzw. in einen Blutplasmakreislauf einzubringen, ist unter einer biologischen Flüssigkeit auch verdünntes Blut bzw. verdünntes Blutplasma zu verstehen. Die Erfindung ist hauptsächlich für den humanmedizinischen Bereich gedacht und bezieht sich daher primär auf menschliche biologische Flüssigkeiten. Dies schließt jedoch nicht aus, dass die Erfindung auch für den veterinärmedizinischen Bereich geeignet ist.

Der Ausdruck "poröses, neutrales, hydrophobes Polymer" bezeichnet einen porösen, wasserunlöslichen Feststoff definierter mittlerer Porengröße. Es kann sich sowohl um ein Homopolymer als auch um ein Heteropolymer handeln. Das Polymer weist äußere und innere Oberflächen auf, welche neutral und hydrophob sind. Der Begriff "neutral" bedeutet nichtionisch. Unter dem Begriff "hydrophob" ist zu verstehen, dass es sich um wasserabweisende Materialstrukturen handelt. Obwohl einem Fachmann auf dem Gebiet bekannt ist, was unter dem Begriff "mittlere Porengröße eines Polymers" zu verstehen ist und wie die Porösität bzw. die mittlere Porengröße gezielt einstellt werden kann, soll dieser Begriff aus Gründen der Klarheit an dieser Stelle dennoch kurz definiert werden. Die mittlere Porengröße bezieht sich auf den mittleren Durchmesser der Poren. Bei einer gaußschen Größenverteilung der Porendurchmesser eines porösen Materials ist der mittlere Porendurchmesser jener, welchem dem Maximum der Verteilungskurve entspricht. Der mittlere Porendurchmesser kann beispielsweise mittels Stickstoffadsorption [wie beschrieben in Weber et al. 2008. Neutral styrene divinylbenzene copolymers for adsorption of toxins in liver failure. Biomacromolecules 9(4):1322-1328] oder mittels Quecksilberintrusion bestimmt werden. Eingestellt wird die Porengröße durch Variation der Konzentration der beteiligten Monomere, des Lösungsmittels oder des Modulators. Je kleiner die Poren des Polymers gewählt sind, umso größer wird die innere Oberfläche des Polymers, die für eine Sorption, insbesondere Adsorption, zur Verfügung steht. Je größer die Poren, umso besser wird die Zugänglichkeit der Poren für größere Moleküle. Ein Herstellungsverfahren eines Polymers definierter Porengröße, wie es für die Erfindung zur Anwendung kommen kann, wurde z.B. von Weber et al. beschrieben [Weber et al. 2008. Neutral styrene divinylbenzene copolymers for adsorption of toxins in liver failure. Biomacromolecules 9(4):1322-1328]. Die Erfindung ist vor allem auf partikelförmige Polymere ausgerichtet. Es wäre auch denkbar, dass das Sorptionsmittel die Form eines Membranfilters hat.

Die äußeren und inneren Oberflächen des porösen, neutralen, hydrophoben Polymers sind mit einer Polypeptidschicht beschichtet, wobei die Polypeptidschicht durch Adsorption von Polypeptidmolekülen an den Oberflächen des Polymers gebildet wird. Der Begriff "Polypeptid" bezieht sich hier auf ein Polypeptid bzw. ein Protein, welches hydrophobe und hydrophile Seitenkette bzw. Domänen aufweist. Der Begriff Polypeptid bezieht sich insbesondere auf Polypeptide die aus mehr als 10 Aminosäuren aufgebaut sind, wobei die Anzahl der Aminosäuren, aus welchen das Polypeptidmolekül aufgebaut ist, nach oben hin offen ist. Insbesondere bezieht sich der Begriff Polypeptid auf Proteine. Beim adsorptiven Beschichten des neutralen, hydrophoben Polymers mit einer Polypeptidlösung treten die hydrophoben Seitenketten bzw. Domänen des Polypeptids (bzw. Proteins) mit der neutralen, hydrophoben Oberfläche des Polymers aufgrund der hydrophoben Interaktion in Wechselwirkung. Die hydrophobe Interaktion, auch hydrophobe Wechselwirkung genannt, besitzt große biochemische Bedeutung und beruht auf dem Phänomen, dass hydrophobe Moleküle in einer polaren Umgebung zur Assoziation neigen. Die hydrophobe Interaktion ist daher keine Kraft per se, sondern wird durch eine polare Umgebung erzwungen. Die hydrophilen Seitenketten des Polypeptids sind nach außen, von der hydrophoben Oberfläche des Polymers weggerichtet. Das Polymer wird durch Auskleiden seiner äußeren und seiner inneren Oberflächen mit der Polypeptidschicht hydrophilisiert. Üblicherweise wird eine Polypeptid-Monoschicht gebildet. Besonders zweckmäßig ist es, wenn die Polypeptidschicht funktionell inert ist, wobei unter dem Begriff "funktionell inert" zu verstehen ist, dass keine spezifische Wechselwirkung zwischen der Polypeptidschicht und den in der biologischen Flüssigkeit vorliegenden Molekülen (z.B. Blutplasmaproteine etc.) stattfindet. Weiters ist es zweckmäßig, wenn die Polypeptidschicht auch nicht unspezifisch mit den in der biologischen Flüssigkeit vorliegenden Molekülen interagiert. Die Eignung eines Polypeptids für die vorliegende Erfindung kann von einem Fachmann im Rahmen von Routineversuchen festgestellt werden.

Es hat sich gezeigt, dass das Protein C durch ein erfindungsgemäßes Sorptionsmittel mit einer mittleren Porengröße von 15 -20 nm nach einiger Zeit aus der biologischen Flüssigkeit entfernt wird, nämlich dann, wenn die biologische Flüssigkeit mit dem Sorptionsmittel ausreichend lange (länger als 15 min) in Kontakt gebracht wurde. Im Vergleich zum erfindungsgemäßen Sorptionsmittel, wird Protein C von einem unbeschichteten neutralen, hydrophoben Polymer jedoch sofort eliminiert. Ein Polymer mit einer mittleren Porengröße von 15-20 nm ist dennoch sehr gut für Leberunterstützungssysteme mit hohen Adsorptionsgeschwindigkeiten für stark proteingebundene Substanzen, z.B. ein Microspheres-based Detoxificaton System (MDS), geeignet. Die Zeit, in welcher die biologische Flüssigkeit mit dem Sorptionsmittel in Kontakt ist, kann bei MDS ausreichend kurz (unter 5 min) gehalten werden, so dass noch physiologisch relevante Mengen an Protein C in der biologischen Flüssigkeit verbleiben. Der Vorteil einer Porengröße von 15-20 nm ist die größere innere Oberfläche und die damit einhergehende bessere Sorptionsleistung für proteingebundene Substanzen.

Bei Verfahren, in welchen die biologische Flüssigkeit länger (länger als 15 min) mit dem erfindungsgemäßen Sorptionsmittel in Kontakt ist, empfiehlt es sich, ein Polymer mit einer mittleren Porengröße größer als 15-20 nm zu verwenden. Es konnte gezeigt werden, dass mit steigender mittlerer Porengröße die Bindung des Protein C an das Polymer weiter gesenkt wird. Bei höheren Porengrößen (≥ 30 nm) ist es auch möglich, die biologische Flüssigkeit für einen sehr langen Zeitraum mit dem erfindungsgemäßen Sorptionsmittel in Kontakt zu bringen, ohne dass die Protein C-Konzentration auf einen physiologisch kritischen Wert sinkt. Bei sehr hohen mittleren Porengrößen von 80-100 nm wird Protein C kaum mehr adsorbiert. Aufgrund dieser Beobachtung ist die mittlere Porengröße des zu beschichtenden neutralen, hydrophoben Polymers nicht kleiner als 15 nm und vorzugsweise größer (≥ 30 nm) zu wählen. Proteingebundene Substanzen wie Bilirubin werden bei Porengrößen von 15 nm oder größer sehr gut adsorbiert.

Die Verwendung Albumin-beschichteter Polymere mit kleinen mittleren Porengrößen von 8 bzw. 9 nm (siehe oben beschriebenen Stand der Technik) erhöht zwar die Biokompatibilität in Bezug auf eine verminderte Bindung der Thrombozyten an den Adsorber während einer Hämoperfusion, bietet jedoch in Bezug auf eine Verminderung der unerwünschten Protein C-Bindung keine zufriedenstellende Lösung. Die bekannten Albumin-beschichteten Polymere wurden bislang mit der Gerinnungsproblematik hinsichtlich des Proteins C nicht in Verbindung gebracht. Darüber hinaus wurde, wie im Stand der Technik bereits erwähnt, mit Albumin-beschichteten Polymeren mit einer mittleren Porengröße von 5-10 nm nachteiligerweise eine verminderte Bilirubin-Adsorption im Vergleich zum unbeschichteten Polymer festgestellt.

Eine mögliche Erklärung für die mit steigender Porengröße sinkende Protein C-Bindung durch das Sorptionsmittel könnte die Folgende sein: möglicherweise ist die adsorptive Beschichtung eines Polymers kleinerer mittlerer Porengröße (15-20 nm) mit einer Polypeptidschicht, insbesondere auf der inneren Polymeroberfläche, aufgrund der Größe des verwendeten Polypeptids (z.B. Albumin) nicht vollständig. Somit kann das relativ kleine Protein C in die Poren rutschen und an den verbleibenden hydrophoben Flächen adsorbiert werden. Bei höheren mittleren Porengrößen (≥ 30 nm) ist eine bessere Zugänglichkeit für das Polypeptid und daher auch eine bessere hydrophile Auskleidung der Polymeroberfläche gegeben. Für Protein C stünden zur Bindung folglich keine hydrophoben Flächen mehr zur Verfügung. Darüber hinaus wird das Protein C durch die hydrophilen Seitenketten bzw. Domänen der Polypeptidschicht abgestoßen. Da die Sorptionsleistung für proteingebundene Substanzen durch die Polypeptidbeschichtung nicht negativ beeinflusst wird, ist anzunehmen, dass am beschichteten Polymer mit höherer Porengröße (≥ 30 nm) noch genügend hydrophobe - allerdings für Protein C nicht zugängliche - Flächen auf der inneren Oberfläche zur Bindung proteingebundener Substanzen zur Verfügung stehen. Die Proteinschicht bildet vermutlich eine Monoschicht aus, die winzig kleine Poren (Picoporen) zwischen den einzelnen Polypeptidmolekülen aufweist, durch welche proteingebundene Substanzen wie Bilirubin durchtreten können und daher trotz Polypeptidbeschichtung noch adsorbiert werden.

Obwohl die Bindung von Protein C bei einer höher gewählten, mittleren Porengröße des unbeschichteten Polymers bis auf ein Minimum gesenkt werden kann, ist es für die klinische Anwendung günstig, wenn die mittlere Porengröße nicht größer als 80 nm ist. Die innere Oberfläche des Polymers würde ansonsten zu klein werden. Die Folge ist eine Verminderung der Sorptionseffizienz (Adsorptionseffizienz) stark proteingebundener Substanzen. Bei Hochrisikopatienten mit Leberversagen ist es empfehlenswert, wenn die mittlere Porengröße nicht größer als 40 nm gewählt ist.

Bei einer besonders bevorzugten Variante weist das unbeschichtete neutrale, hydrophobe Polymer eine mittlere Porengröße von 30 bis 40 nm auf. Es hat sich gezeigt, dass bei dieser Variante sehr wenig Protein C adsorbiert wird, auch wenn die biologische Flüssigkeit mit dem Sorptionsmittel über einen langen Zeitraum in Kontakt gebracht wird. Darüber hinaus ist die innere Oberfläche des Polymers bei dieser Porengröße noch so groß, dass eine hocheffiziente Adsorption proteingebundener Substanzen gewährleistet ist.

Mit Vorteil kommen neutrale, hydrophobe Polymere zum Einsatz, die sich in der klinischen Praxis bereits etabliert haben. Besonders bevorzugt kommt ein Styrol/Divinylbenzol-Copolymer zum Einsatz. Styrol/Divinylbenzol-Copolymere haben sich für die Adsorption von Toxinen des Leber- und Nierenversagens bewährt und zeichnen sich durch eine gute Bioverträglichkeit, Ungiftigkeit, sowie gute Stabilität und Sterilisierbarkeit aus. Darüber hinaus ist die Porengröße leicht und reproduzierbar einstellbar. Ferner können sehr kleine Partikelgrößen eingestellt werden. Es ist jedoch auch möglich, neutrale, hydrophobe Polymere mit einer einem Styrol/Divinylbenzol-Copolymer ähnlichen Struktur zu verwenden; diese sollten ähnliche Wirkung wie ein Styrol/Divinylbenzol-Copolymer zeigen. Für einen Fachmann ist es möglich, in wenigen Routineversuchen festzustellen, ob ein neutrales, hydrophobes Polymers für die Durchführung der Erfindung geeignet ist.

Zum Beschichten des neutralen, hydrophoben Polymers mit einer Polypeptidschicht werden vorzugsweise Polypeptide mit einem relativen Molekulargewicht von 40 000 bis 80 000 Da bzw. einer Größendimension von bis zu ungefähr 10 nm x 10 nm x 10 nm verwendet.

Die Polypeptidschicht kann aus zumindest einer Art von Polypeptid bestehen, wobei die zumindest eine Art von Polypeptid gewählt ist aus Albumin, insbesondere Humanalbumin, und Albumin-ähnlichen Polypeptiden. Albumin-ähnliche Polypeptide sind dem Albumin hinsichtlich ihrer Struktur ähnlich bzw. weisen Albumin-ähnliche Eigenschaften auf. Albumin-ähnliche Polypeptide rekrutieren sich u.a. aus Subtypen des Albumins, wie beispielsweise Präalbumin. Die Albumin-ähnlichen Polypeptide können entweder aus natürlichen Quellen stammen oder synthetisch hergestellt werden. Die Polypeptidschicht kann aus einer einzigen Art von Polypeptid oder aus einer Mischung von zwei oder mehr Polypeptidarten bestehen. Das Polypeptid kann sowohl aus einer natürlichen Quelle stammen als auch rekombinant hergestellt sein. Die Eignung eines Polypeptids oder einer Mischung von zwei oder mehr Polypeptidarten für die Durchführung der Erfindung kann von einem Fachmann im Rahmen von Routineversuchen festgestellt werden.

Es sei an dieser Stelle nochmals erwähnt, dass die Bildung der Polypeptidschicht auf den inneren und äußeren Oberflächen des neutralen, hydrophoben Polymers aufgrund einer hydrophoben Interaktion zwischen den hydrophoben Seitenketten des verwendeten Polypeptids bzw. Proteins und der hydrophoben Oberfläche des Polymers beruht.

Aufgrund der ausgezeichneten Biokompatibilität und der Verfügbarkeit in hoher Reinheit ist die Verwendung von Humanalbumin (Humanes Serum Albumin, HSA) zum Beschichten des Polymers besonders günstig.

Die Erfindung bezieht sich ferner auf ein Verfahren zum Herstellen eines erfindungsgemäßen Sorptionsmittels, bei welchem zum adsorptiven Beschichten des neutralen, hydrophoben Polymers mit der Polypeptidschicht eine bis zu 10%ige Polypeptidlösung verwendet wird.

Bei einer besonders bevorzugten Variante handelt es sich bei der Polypeptidlösung um eine Humanalbuminlösung. Wie oben bereits detailliert beschrieben könnten weitere vorteilhafte Polypeptide gewählt sein aus Albumin, insbesondere Humanalbumin, und Albuminähnlichen Polypeptiden. Darüber hinaus kann die Polypeptidlösung auch mehr als eine Art eines Polypeptids enthalten.

Es konnte gezeigt werden, dass bereits eine 1%ige Polypeptidlösung ausreicht, um ein Sorptionsmittel mit den erfindungsgemäßen Eigenschaften bereitzustellen. Die Verwendung einer höher konzentrierten Polypeptidlösung (5-10%) führt zu keinen signifikanten Unterschieden oder Verbesserungen hinsichtlich der Protein C-Bindung. Aus ökonomischen Gründen - hochreine Polypeptidlösungen sind teuer - wird daher bei einer ersten vorteilhaften Ausführung des Verfahrens eine 1%ige Polypeptidlösung zum Beschichten des neutralen, hydrophoben Polymers verwendet.

Bei einer weiteren vorteilhaften Ausführung des Verfahrens wird eine 10%ige Polypeptidlösung verwendet. Das adsorptive Beschichten des Polymers mit einer höher konzentrierten Polypeptidlösung (10%ig) hat den Vorteil, dass nicht nur die unerwünschte Protein C-Bindung an das Sorptionsmittel, sondern auch die Fibrinogen-Bindung deutlich gesenkt werden kann.

Der pH-Wert der Polypeptidlösung wird vorzugsweise auf 7.40 eingestellt. Dies hat den Vorteil, dass bereits beim adsorptiven Beschichten des Trägers (Polymers) mit dem Polypeptid ein pH-Wert, welcher dem physiologischen pH-Wert des Blutes entspricht, verwendet wird. Dies hat nicht nur Vorteile hinsichtlich der Patientensicherheit (Vermeidung einer Azidose bzw. Alkalose), sondern auch Vorteile hinsichtlich der Herstellung, da umständliche Waschvorgänge zum Einstellen eines physiologischen pH-Werts (das Sorptionsmittel liegt meistens in Form einer Suspension vor) nicht notwendig sind.

Es kann sich auch als günstig erweisen, wenn der pH-Wert nahe dem Isoelektrischen Punkt des Polypeptids gewählt ist. Es ist bekannt, dass die Belegungsdichte des Polypeptids am Polymer durch Variieren des pH-Werts veränderbar ist [Ton et al. 1979. Adsorption of Human Serum Albumin to Amberlite XAD-7 Resin. J Biomed Mater Res 13:407-422]. Die Konzentration der Polypeptidlösung kann durch Optimieren des pH-Werts sehr gering gehalten werden, wodurch Herstellungskosten eingespart werden können. Das Optimieren des pH-Werts kann im Rahmen von Routineversuchen erfolgen.

Neben den physikochemischen Eigenschaften der Polymeroberfläche und der mittleren Porengröße spielt auch die Partikelgröße des Polymers eine Rolle beim Adsorptionsvorgang. Die Partikelgröße beeinflusst die Kinetik der Adsorption. Die Adsorption hängt im Wesentlichen von der Diffusionszeit (und somit auch von der Diffusionsgeschwindigkeit) ab, die wiederum als Quadrat proportional zur Entfernung ist. Bei klein gewählter Partikelgröße sind die Diffusionswege sehr klein, wodurch die Geschwindigkeit der Elimination umgekehrt proportional zur Partikelgröße ist. Darüber hinaus liegt bei einer kleinen Partikelgröße ein großes Oberflächen/Volumen-Verhältnis vor. Um die Sorptionsgeschwindigkeit und die Sorptionskapazität für stark proteingebundene Substanzen so groß wie möglich zu gestalten, ist es von Vorteil, wenn das Sorptionsmittel aus Mikropartikeln besteht, wobei die Mikropartikel eine Partikelgröße von 20 µm oder kleiner aufweisen.

Die Mikropartikel finden insbesondere in MDS (Microspheres-based Detoxificaton System) Anwendung, welches weiter oben im Stand der Technik bereits erwähnt wurde. Die Mikropartikel zirkulieren als Suspension in einem Reinigungskreislauf (Plasmakreislauf) auf der Filtratseite eines Membranfilters. Sollte allerdings der Membranfilter undicht werden, besteht die Gefahr, dass Mikropartikel in den extrakorporalen Blutkreislauf und weiter in den Körper des Patienten gelangen und dort zu einer Lungenembolie führen. Aus diesem Grunde ist es bei einer besonders vorteilhaften Variante vorgesehen, dass die Mikropartikel eine Partikelgröße von 8 µm oder kleiner, idealerweise 5 µm oder kleiner, aufweisen, da bei diesen kleinen Partikelgrößen die Gefahr einer Lungenembolie umgangen werden kann.

Das erfindungsgemäße Sorptionsmittel ist weiters ein geeignetes Füllmaterial für eine Sorptionseinrichtung, beispielsweise eine Adsorbtionssäule.

Die Erfindung bezieht sich weiters auf ein Verfahren zum Entfernen proteingebundener Substanzen aus einer biologischen Flüssigkeit, wobei eine proteingebundene Substanzen enthaltende biologische Flüssigkeit mit einem erfindungsgemäßen Sorptionsmittel in Kontakt gebracht wird. Dies kann vor allem im Rahmen einer extrakorporalen Leberunterstützung erfolgen, bei welcher Blut oder Blutplasma mittels des Sorptionsmittels von proteingebundenen Substanzen gereinigt wird. Eine extrakorporale Leberunterstützung kommt bei Patienten mit Leberversagen bzw. verminderter Leberfunktion sowie bei Patienten mit Niereninsuffizienz zur Anwendung. Wie oben beschrieben kann die biologische Flüssigkeit eine Sorptionseinrichtung, welche das Sorptionsmittel enthält, passieren. Das Sorptionsmittel kann aber auch in der biologischen Flüssigkeit suspendiert sein, wie dies in einem MDS zur Anwendung kommt.

Die Erfindung bezieht sich ferner auf die Verwendung des erfindungsgemäßen Sorptionsmittels als selektives Sorptionsmittel zum Entfernen proteingebundener Substanzen aus einer biologischen Flüssigkeit, wobei es sich bei den proteingebundenen Substanzen insbesondere um Bilirubin, aromatische Aminosäuren, Gallensäuren, Phenole und Merkaptane handeln kann. Der Begriff "proteingebundene Substanzen" bezieht sich primär auf proteingebundene toxische Stoffe (sogenannte Lebergifte) die sich bei Patienten mit Leberfunktionsstörungen bzw. Leberversagen im Blut anreichern. Die verbesserten Selektivitätseigenschaften des erfindungsgemäßen Sorptionsmittels beruhen auf einer deutlichen Verminderung hinsichtlich der Protein C-Bindung durch das erfindungsgemäße Sorptionsmittel, während die Sorptionsleistung für proteingebundene Substanzen im Vergleich zum ungeschichteten Polymer nicht beeinträchtigt wird.

Die Erfindung bezieht sich außerdem auf die Verwendung des erfindungsgemäßen Sorptionsmittels als selektives Sorptionsmittel zum Entfernen proteingebundener Substanzen aus einer zellfreien biologischen Flüssigkeit, insbesondere Blutplasma.

Die Erfindung bezieht sich ferner auf die Verwendung des erfindungsgemäßen Sorptionsmittels als selektives Sorptionsmittel zum Entfernen proteingebundener Substanzen aus einer Zellen enthaltenden, biologischen Flüssigkeit, insbesondere Blut.

Die vorliegende Erfindung wird im Folgenden anhand von nicht einschränkenden Beispielen näher erläutert.

### BEISPIEL 1: Herstellen Albumin-beschichteter Styrol/Divinylbenzol-Copolymere

Die zum Beschichten verwendeten Styrol-Divinylbenzol-Copolymere unterschiedlicher mittlerer Porengröße sind in der Tabelle 1 aufgelistet, wobei die Partikelgröße 5µm +/- 3-4 µm beträgt:

**Tabelle 1: Styrol-Divinylbenzol-Copolymere**

| Bezeichnung des Styrol-Divinylbenzol-Copolymers | Mittlere Porengröße (nm) |
|---|---|
| # 1822 | 15-20 |
| # 1824 | 30-40 |
| # 1825 | 80-100 |

Zunächst wird eine 50%ige Suspension eines Styrol/Divinylbenzol-Copolymers definierter mittlerer Porengröße in physiologischer NaCl-Lösung hergestellt Davon wird die doppelte Ansatzmenge in ein 15 ml Greinerröhrchen pipettiert und abzentrifugiert. Der Überstand wird abgehoben. Das verbleibende Styrol/Divinylbenzol-Copolymer entspricht dem Ansatzvolumen.

Anschließend wird das Styrol/Divinylbenzol-Copolymer mit einer Humanalbuminschicht durch Adsorbieren von Humanalbumin aus einer Humanalbuminlösung an die äußeren und inneren Oberflächen des Copolymers beschichtet Im Doppelansatz wird je ein Röhrchen mit 1 ml Copolymer und 5 ml Humanalbuminlösung (Humanes Serum Albumin, HSA) (Humanes Albumin Octapharma 20%ige Lösung, Lot: B17B6331) versetzt Die Konzentration der Humanalbuminlösung kann 1-10%ig sein. Die Röhrchen werden im Schüttler (Enviro Genie®) bei 10°C, Frequenz 25:50 für 21 h inkubiert. Als Kontrolle wird je ein Röhrchen mit Copolymer und 0.9%iger NaCl-Lösung mitgeführt. Ein Röhrchen wird als Negativkontrolle nur mit gespiketem Plasma (siehe Abschnitt 2.2.1.)ohne Copolymer mitgeführt. Nach dem Ende der Inkubationszeit im Schüttler (21h) werden die Röhrchen zentrifugiert und der Überstand abgekippt.

### BEISPIEL 2: Austesten Humanalbumin-beschichteter Styrol/Divinylbenzol-Copolymere unterschiedlicher Porengröße hinsichtlich der Adsorption proteingebundener Substanzen und der Protein C-Adsorption im Vergleich mit unbeschichteten Styrol/Divinylbenzol-Copolymeren.

### 2.1. Adsorber

Zunächst wurden Styrol/Divinylbenzol-Copolymere unterschiedlicher Porengröße bereitgestellt. Die Partikelgröße der Styrol/Divinylbenzol-Copolymere betrug 5µm +/- 3-4 µm. Die Styrol/Divinylbenzol-Copolymere wurden anschließend mit einer 10%igen Humanalbuminlösung (HSA) gemäß dem Herstellungsprotokoll aus Beispiel 1 beschichtet. Die Adsorber sind in Tabelle 2 aufgelistet:

**Tabelle 2: Adsorber (Styrol/Divinylbenzol-Copolymer) unterschiedlicher Porengröße mit und ohne HSA-Beschichtung**

| ADSORBERBEZEICHNUNG | Mittlere Porengröße (nm) |
|---|---|
| # 1822 mit HSA | 15-20 |
| # 1822 ohne HSA | 15-20 |
| # 1824 mit HSA | 30-40 |
| # 1824 ohne HSA | 30-40 |
| # 1825 mit HSA | 80-100 |
| # 1825 ohne HSA | 80-100 |

Die Bezeichnung ""mit HSA" bezeichnet Adsorber, die mit Albumin beschichtet ist, "ohne HSA" bezeichnet den jeweiligen unbeschichteten Adsorber (= Styrol/Divinylbenzol-Copolymer ohne HSA-Beschichtung).

### 22. Batchtest

Die oben aufgelisteten Adsorber wurden in einem Batchtest auf ihre Eigenschaften hinsichtlich der Adsorption proteingebundener Substanzen sowie hinsichtlich Ihrer Protein-C-Adsorption aus Humanplasma untersucht. Für diese Versuche wurden die Adsorber mit gespiketem Plasma (siehe Abschnitt 2.2.1.) überschichtet.

### 2.2.1. Herstellung eines Plasma Spikes:

Für die Herstellung eines Plasmaspikes wurde Blutplasma aus einem gesunden Spender mit einer definierten Konzentration an Lebertoxinen gespikt. 100 ml gespiketes Plasma enthielt Lebertoxine in den folgenden Konzentrationen: 300 µM Bilirubin, 100 µM Cholsäure, 100 µM Tryptophan, 2 mM Phenol.

Die Lebertoxine wurden wie folgt verarbeitet:

Für den Bilirubin-Ansatz wurden 0,0175 g Bilirubin eingewogen und in 2 ml 0,3 N NaOH aufgelöst.

Für den gemischten Toxinansatz wurden 0,02043 g Cholsäure, 0,0102 g Tryptophan und 0,0940 g Phenol eingewogen und gemeinsam in insgesamt 5 ml 0,3 N NaOH gerührt.

Für 100 ml gespiketes Plasma wurden 2 ml des Bilirubin-Ansatzes und 1 ml des gemischten Toxinansatzes zu 96 ml Plasma zugesetzt und 60 min langsam und lichtgeschützt gerührt. Nach 60 min wurden 3 ml 0,3 N HCl zugegeben.

Die in Tabelle 2 aufgelisteten Adsorber wurden in Teströhrchen mit gespiketem Plasma überschichtet, wobei 1 ml Adsorber mit 9 ml gespiktem Plasma versetzt wurde. Dies entspricht einem 10%igen Adsorberansatz. Als Negativkontrolle wurde ein Teströhrchen mit gespiketem Plasma ohne Adsorber mitgeführt Die Teströhrchen wurden 1 h bei 37°C am Schüttler Enviro Genie®, Frequenz 25:50, inkubiert

### 2.2.2. Probenahmen:

Nach 5, 15, 30 und 60 min wurde pro Teströhrchen jeweils 1 ml entnommen und sofort 10 min bei 11000g zentrifugiert. Der Überstand wurde sofort in ein Eppendorf-Reaktionsgefäß überführt Für die Messung zum Zeitpunkt 0 min wurde die Negativkontrolle ohne Adsorber (als "Kontrolle Plasma" bezeichnet) herangezogen. Die Proben wurden bis zur Analytik bei -20 °C lichtgeschützt gelagert.

### 2.2.3. Analytik:

Es wurden sowohl die zugesetzten Lebergifte (Bilirubin, Cholsäure, Tryptophan, Phenol) sowie die Adsorption von den im Plasma vorliegenden Proteinen Albumin und Protein C analysiert:
Bilirubin, Kit Lot: 14652700 (Roche)
Cholsäure, Lot: R284067 (Roche)
Tryptophan Kit Lot: 685125-01 (Roche)
Phenol: HPLC-Chromatographie
Albumin Kit Lot: 688271-01 (Roche)
Protein C ELISA Kit Lot: 12021228 (Technoclone)
Die Analytik erfolgte an einem Hitachi 902 (VT).

### 2.3. Versuchsergebnisse:

Die Ergebnisse der Versuche sind in den Figuren in Form von Diagrammen dargestellt. Die Figuren zeigen in:
**Fig. 1A** die Adsorption des Bilirubins (Bilirubinkonzentration im Überstand in mg/dL) durch die HSA-beschichteten Adsorber #*1822 mit HSA* und #*1824 mit HSA* im Vergleich mit den jeweiligen unbeschichteten Adsorbern #*1822 ohne HSA* und #*1824 ohne HSA* im Zeitverlauf [min],
**Fig. 1B** die Adsorption des Bilirubins (Bilirubinkonzentration im Überstand in mg/dL) durch den HSA-beschichteten Adsorber #*1825 mit HSA* im Vergleich mit dem unbeschichteten Adsorber #*1825 ohne HSA* im Zeitverlauf [min],
**Fig. 2A** die Adsorption der Cholsäure (Cholsäurekonzentration im Überstand in µmol/L) durch die HSA-beschichteten Adsorber #*1822 mit HSA* und #*1824 mit HSA* im Vergleich mit den jeweiligen unbeschichteten Adsorbern #*1822 ohne HSA* und #*1824 ohne HSA* im Zeitverlauf [min],
**Fig. 2B** die Adsorption der Cholsäure (Cholsäurekonzentration im Überstand in µmol/L) durch den HSA-beschichteten Adsorber #*1825 mit HSA* im Vergleich mit dem unbeschichteten Adsorber #*1825 ohne HSA* im Zeitverlauf [min],
**Fig. 3A** die Adsorption des Tryptophans (Tryptophankonzentration im Überstand in µmol/L) durch die HSA-beschichteten Adsorber #*1822 mit HSA* und #*1824 mit HSA* im Vergleich mit den jeweiligen unbeschichteten Adsorbern #*1822 ohne HSA* und #*1824 ohne HSA* im Zeitverlauf [min],
**Fig. 3B** die Adsorption des Tryptophans (Tryptophankonzentration im Überstand in µmol/L) durch den HSA-beschichteten Adsorber #*1825 mit HSA* im Vergleich mit dem unbeschichteten Adsorber #*1825 ohne HSA* im Zeitverlauf [min],
**Fig. 4A** die Adsorption des Phenols (Phenolkonzentration im Überstand in mmol/L) durch die HSA-beschichteten Adsorber #*1822 mit HSA* und #*1824 mit HSA* im Vergleich mit den jeweiligen unbeschichteten Adsorbern #*1822 ohne HSA* und #*1824 ohne HSA* im Zeitverlauf [min],
**Fig. 4B** die Adsorption des Phenols (Phenolkonzentration im Überstand in mmol/L) durch den HSA-beschichteten Adsorber #*1825 mit HSA* im Vergleich mit dem unbeschichteten Adsorber #*1825 ohne HSA* im Zeitverlauf [min],
**Fig. 5A** die Adsorption des Plasmaproteins Albumin (Albuminkonzentration im Überstand in g/dL) durch die HSA-beschichteten Adsorber #*1822 mit HSA* und #*1824 mit HSA* im Vergleich mit den jeweiligen unbeschichteten Adsorbern #*1822 ohne HSA* und #*1824 ohne HSA* im Zeitverlauf [min],
**Fig. 5B** die Adsorption des Plasmaproteins Albumin (Albuminkonzentration im Überstand in g/dL) durch den HSA-beschichteten Adsorber #*1825 mit HSA* im Vergleich mit dem unbeschichteten Adsorber #*1825 ohne HSA* im Zeitverlauf [min], und
**Fig. 6** die Adsorption des Gerinnungsproteins Protein C (in %) durch die erfindungsgemäßen HSA-beschichteten Adsorber #*1822 mit HSA,* #*1824 mit HSA* und #*1825 mit HSA* im Vergleich mit den jeweiligen unbeschichteten Adsorbern #*1822 ohne HSA,* #*1824 ohne HSA* und #*1825 ohne HSA* nach 0, 5,15 und 60 min Inkubation.

### 2.4. Zusammenfassung der Ergebnisse aus dem Batchtest:

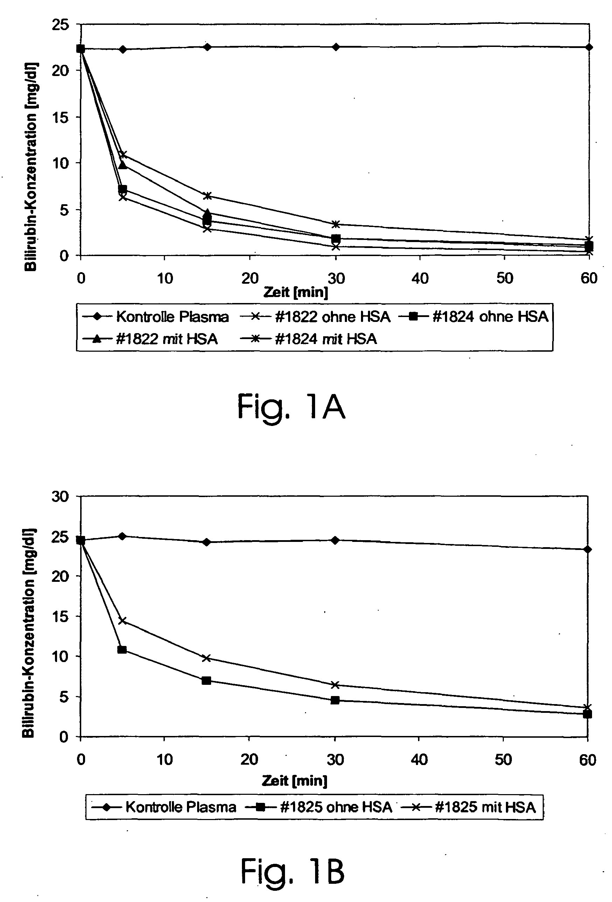

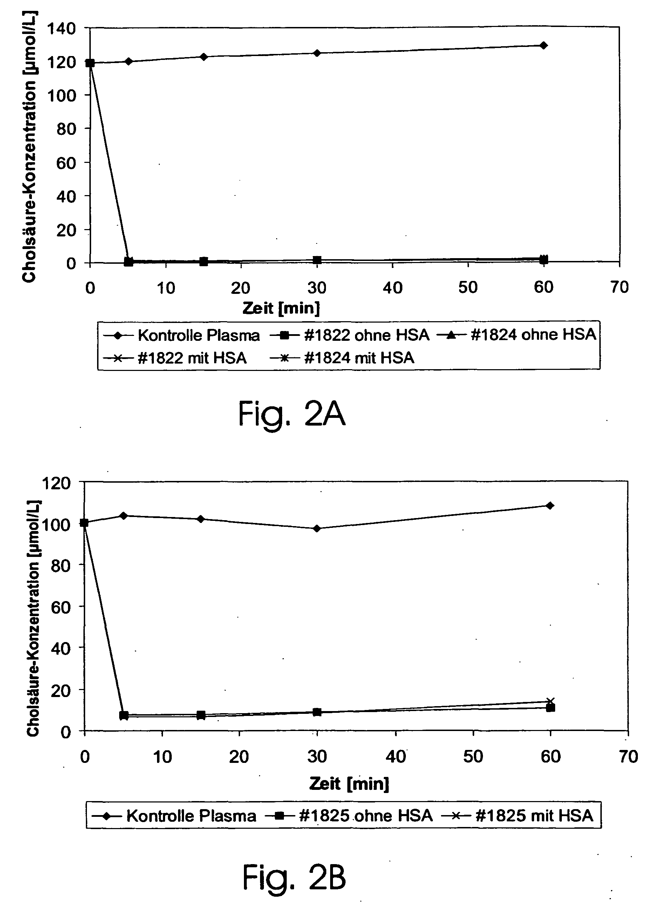

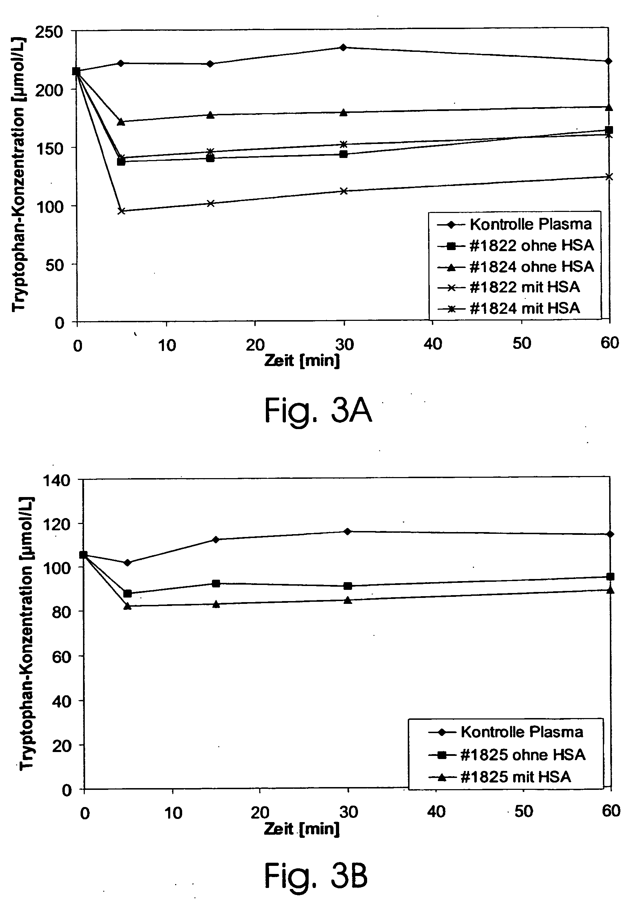

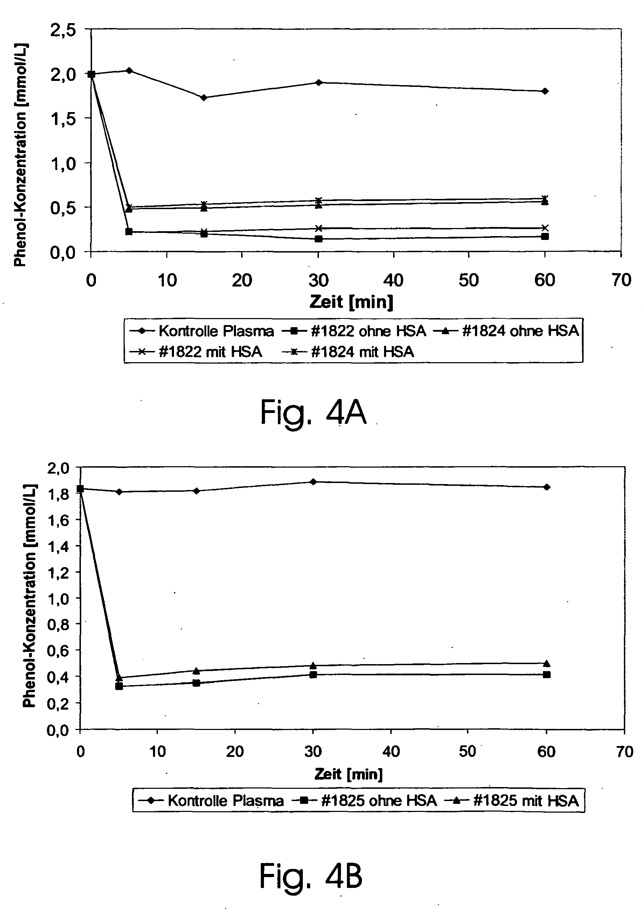

Die Ergebnisse des Batchtests zeigen, dass durch die Albuminbeschichtung des Adsorbers die Biokompatibilität deutlich verbessert werden kann, insbesondere dadurch, dass die Adsorption des natürlichen Antikoagulans Protein C verhindert oder stark reduziert wird. Es ist somit davon auszugehen, dass dies auch für das Protein S zutrifft Dies ist bei Patienten mit Sepsis oder Leberversagen von großer Bedeutung, da die Gerinnungsfähigkeit des Blutes dieser Patienten gestört ist und nicht zusätzlich beeinträchtigt werden sollte. Darüber hinaus wird durch die HSA-Proteinbeschichtung die Charakteristik des Adsorbers hinsichtlich der Adsorption wesentlicher Toxine (siehe Fig. 1A, Fig. 1B, Fig. 2A, Fig. 2B, Fig. 3A, Fig. 3B, Fig. 4A und Fig. 4B) des Leber- und Nierenversagens nicht beeinflusst, sodass sowohl deren Adsorptionskapazität als auch deren Adsorptionskinetik weitgehend erhalten bleiben.

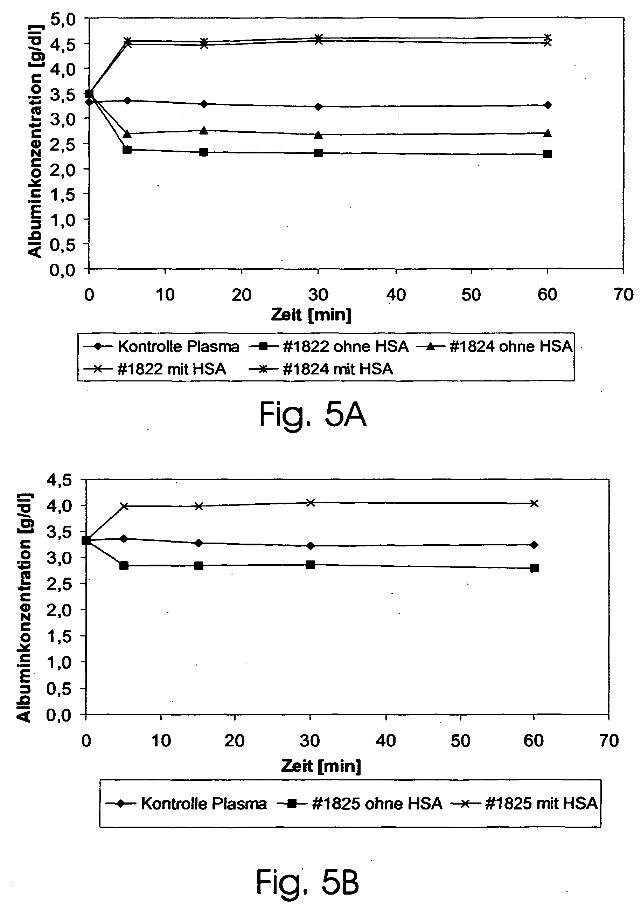

Die geringfügige Desorption des Albumins (siehe Fig. 5A und Fig. 5B) konnte dadurch erklärt werden, dass sich beim Beschichtungsvorgang mehr Albumin als notwendig an das Polymer bindet und eine Doppel- oder Mehrfach-Schicht ausbildet. Das überschüssige Albumin wird unter bestimmten Umständen wieder desorbiert. Für die beschriebene vorteilhafte Funktion der Albuminschicht ist die Albumin-Monoschicht verantwortlich, welche in jedem Fall erhalten bleibt.

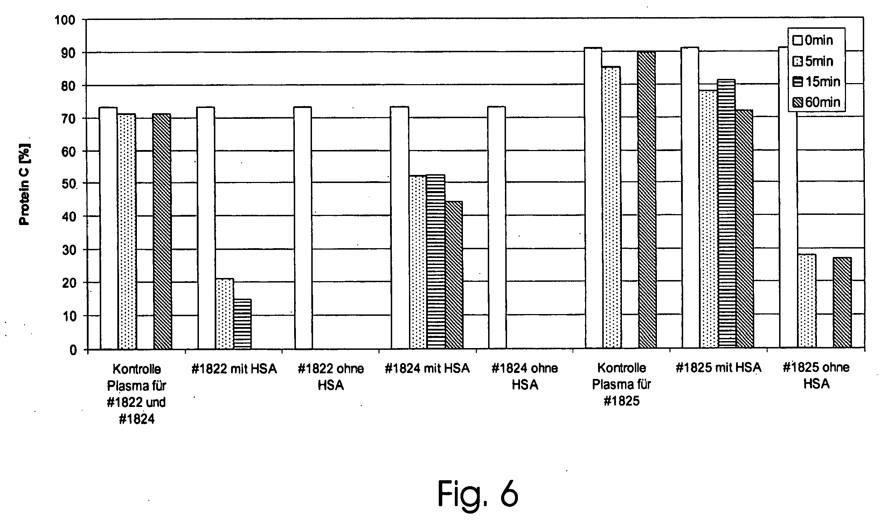

Wie aus der Fig. 6 deutlich zu erkennen ist, konnte durch die adsorptive Beschichtung des Styrol/Divinylbenzol-Copolymers mit einer Humanalbuminschicht eine deutliche Verringerung der Protein C Adsorption erreicht werden. Während beim unbeschichteten Copolymer mit niedrigen mittleren Porengrößen (15-20 nm und 30-40 nm) Protein C gänzlich aus dem Plasma eliminiert wird, verbleibt bei großen mittleren Porengrößen (#1825 ohne HSA, Porengröße 80-100 nm), ein Anteil des Protein C im Plasma. Adsorber mit Porengrößen größer als 80 nm sind für die klinische Anwendung aufgrund der verkleinerten inneren Oberfläche nur mehr bedingt geeignet, z.B. bei Patienten mit nicht lebensbedrohlichen Leberfunktionsstörungen. Im Gegensatz zu den unbeschichteten Adsorbern konnte mit HSA-beschichteten Adsorbern eine deutliche Reduktion der Protein C-Adsorption bereits bei einer Porengröße von 15-20 nm festgestellt werden. Die Protein C-Adsorption der HSA-beschichteten Adsorber sinkt weiter mit ansteigender Porengröße. Besonders günstig ist es, wenn der HSA-beschichtete Adsorber eine Porengröße von 30-40 nm aufweist, da bei dieser Porengröße einerseits wenig Protein C eliminiert wird und andererseits die innere Oberfläche des Adsorbers noch groß genug für eine hocheffiziente Entfernung proteingebundener Substanzen ist. Bei hohen Porengrößen (80-100 nm) wird Protein C kaum mehr adsorbiert, allerdings ist auch - wie erwähnt - die Adsorptionsleistung für proteingebundene Substanzen deutlich geringer.

### BEISPIEL 3: Austesten der Adsorption von proteingebundenen Substanzen und Protein C in Abhängigkeit der Konzentration der Humanalbuminlösung.

Der Einfluss der Konzentration einer Humanalbuminlösung, welche zum Beschichten eines neutralen, hydrophoben Styrol/Divinyl-Copolymers verwendet wird (siehe Protokoll in Beispiel 1), auf die Adsorption von proteingebundenen Substanzen und Protein C wurde ausgetestet. Dieser Versuch hatte den Zweck, die ideale Konzentration der Humanalbuminlösung zum Beschichten des Copolymers auszutesten.

Der unbeschichtete Adsorber # 1824 aus Beispiel 1 (mittlere Porengröße 30-40 nm) wurde gemäß dem Protokoll aus Beispiel 1 mit Humanalbumin beschichtet, wobei zum Beschichten drei verschiedene Konzentrationen der Humanalbuminlösung (1% HSA, 5% HSA und 10% HSA) verwendet wurden. In einem Batchtest gemäß dem Protokoll aus dem Abschnitt 2.2. wurden die HSA-beschichteten Adsorber mit gespiktem Plasma überschichtet. Zu Vergleichszwecken wurde ein Röhrchen mit einem unbeschichteten Adsorber mitgeführt. Weiters wurde als Negativkontrolle ein Röhrchen nur mit gespiktem Plasma ohne Adsorber mitgeführt. Die Analytik wurde ebenfalls gemäß dem Protokoll aus Abschnitt 2.2. durchgeführt.

Die verschiedenen im Batchtest verwendeten Ansätze sind in Tabelle 3 aufgelistet.

**Tabelle 3: Ansätze zum Austesten der Konzentration der Humanalbuminlösung**

| Negativkontrolle Plasma |
|---|
| # 1824 ohne HSA |
| # 1824 1% HSA |
| # 1824 5% HSA |
| # 1824 10% HSA |

### Zusammenfassung der Ergebnisse des Batchtest:

Die Konzentration der HSA-Lösung hatte keinerlei Einfluss auf die Adsorption des Bilirubins, der Cholsäure, des Albumins und des Phenols. Die Kurven sind im Wesentlichen deckungsgleich.

Bei einer HSA-Konzentration von 10% konnte eine geringfügig bessere Tryptophan-Adsorption als bei HSA-Konzentrationen von 1 bzw. 5% festgestellt werden.

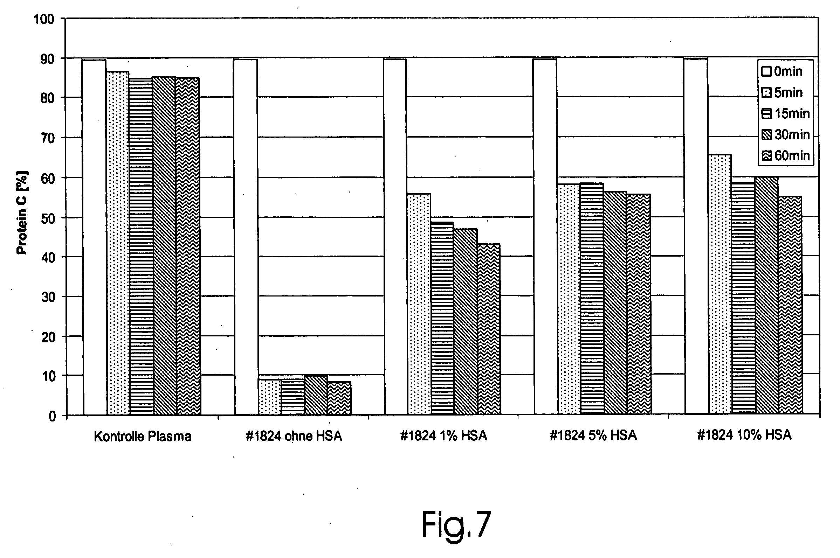

Die Adsorption des Gerinnungsproteins Protein C (in %) durch die mit verschiedenen Humanalbuminlösungen (1%ig, 5%ig und 10%ig) beschichteten Adsorber ist in Abhängigkeit der Zeit (0, 5, 15, 30 und 60 min Inkubation) in der Fig. 7 dargestellt.

Die Protein C Adsorption wird durch die Konzentration der zum Beschichten verwendeten Humanalbuminlösung nur unwesentlich beeinflusst. Es konnte lediglich eine geringfügige Verminderung der Protein C-Adsorption bei steigender Konzentration der Humanalbuminlösung festgestellt werden. Aufgrund dieses Ergebnisses ist eine 1%ige Humanalbuminlösung zum Beschichten des Copolymers völlig ausreichend, um eine Adsorption des Protein C stark zu reduzieren.

Obwohl aus ökonomischen Gründen einer 1%igen Humanalbuminlösung der Vorzug gegeben wird, hat die Verwendung einer 10%igen Humanalbuminlösung zum Beschichten Vorteile hinsichtlich der Adsorption des Fibrinogens. Während bei 1% HSA und 5% HSA Fibrinogen nach einigen Minuten vollständig aus dem Plasma eliminiert war, konnte die Adsorption unter Verwendung eines mit 10% HSA beschichteten Adsorbers deutlich verringert werden.

## Patentansprüche

1. Sorptionsmittel zum Entfernen proteingebundener Substanzen aus einer biologischen Flüssigkeit, **dadurch gekennzeichnet, dass** das Sorptionsmittel (a) ein poröses, neutrales, hydrophobes Polymer und (b) eine am Polymer adsorbierte Polypeptidschicht aufweist, wobei das neutrale, hydrophobe Polymer eine mittlere Porengröße von 30 bis 40 nm aufweist und wobei das Sorptionsmittel aus Mikropartikeln besteht, wobei die Mikropartikel eine Partikelgröße von 20 µm oder kleiner aufweisen.

2. Sorptionsmittel nach Anspruch 1, **dadurch gekennzeichnet, dass** das neutrale, hydrophobe Polymer ein Styrol/Divinylbenzol-Copolymer ist.

3. Sorptionsmittel nach Anspruch 1 oder 2, **dadurch gekennzeichnet, dass** die Polypeptidschicht aus zumindest einer Art von Polypeptid besteht, wobei die zumindest eine Art von Polypeptid gewählt ist aus Albumin, insbesondere Humanalbumin, und Albumin-ähnlichen Polypeptiden.

4. Sorptionsmittel nach einem der Ansprüche 1 bis 3, **dadurch gekennzeichnet, dass** die Mikropartikel eine Partikelgröße von 8 µm oder kleiner aufweisen.

5. Sorptionsmittel nach Anspruch 4, **dadurch gekennzeichnet, dass** die Mikropartikel eine Partikelgröße von 5 µm oder kleiner aufweisen.

6. Sorptionsmittel nach einem der Ansprüche 1 bis 5 zur Behandlung von Leberfunktionsstörungen oder Leberversagen durch Inkontaktbringen des Sorptionsmittels mit einer proteingebundene Substanzen enthaltenden biologischen Flüssigkeit.

7. Sorptionsmittel nach einem der Ansprüche 1 bis 5 zur Behandlung von Niereninsuffizienz durch Inkontaktbringen des Sorptionsmittels mit einer proteingebundene Substanzen enthaltenden biologischen Flüssigkeit.

8. Sorptionsmittel nach Anspruch 6 oder 7, **dadurch gekennzeichnet, dass** die biologische Flüssigkeit eine zellfreie biologische Flüssigkeit, insbesondere Blutplasma, ist.

9. Sorptionsmittel nach Anspruch 6 oder 7, **dadurch gekennzeichnet, dass** die biologische Flüssigkeit eine Zellen enthaltende biologische Flüssigkeit, insbesondere Blut, ist.

10. Sorptionsmittel nach einem der Ansprüche 6 bis 9, **dadurch gekennzeichnet, dass** es sich bei den proteingebundenen Substanzen um Bilirubin, aromatische Aminosäuren, Gallensäuren, Phenole und Merkaptane handelt.

11. Sorptionseinrichtung, enthaltend ein Sorptionsmittel nach einem der Ansprüche 1 bis 10.

12. Plasmakreislauf, enthaltend eine Suspension eines Sorptionsmittels nach einem der Ansprüche 1 bis 10.

13. Microsphere-based Detoxification System (MDS), enthaltend ein Sorptionsmittel nach einem der Ansprüche 1 bis 10.

14. Verfahren zum Herstellen eines Sorptionsmittels zum Entfernen proteingebundener Substanzen aus einer biologischen Flüssigkeit, **dadurch gekennzeichnet, dass** ein poröses, neutrales, hydrophobes Polymer mit einer Polypeptidschicht adsorptiv beschichtet wird, wobei das neutrale, hydrophobe Polymer eine mittlere Porengröße von 30 bis 40 nm aufweist und wobei das Sorptionsmittel aus Mikropartikeln besteht, wobei die Mikropartikel eine Partikelgröße von 20 µm oder kleiner aufweisen, und wobei zum adsorptiven Beschichten des neutralen, hydrophoben Polymers mit der Polypeptidschicht eine bis zu 10%ige Polypeptidlösung verwendet wird.

15. Verfahren nach Anspruch 14, **dadurch gekennzeichnet, dass** zum adsorptiven Beschichten des neutralen, hydrophoben Polymers eine 1%ige Polypeptidlösung verwendet wird.

16. Verfahren nach Anspruch 14, **dadurch gekennzeichnet, dass** zum adsorptiven Beschichten des neutralen, hydrophoben Polymers eine 10%ige Polypeptidlösung verwendet wird.

17. Verfahren nach einem der Ansprüche 14 bis 16, **dadurch gekennzeichnet, dass** die Polypeptidlösung zumindest eine Art von Polypeptid enthält, wobei das Polypeptid gewählt ist aus Albumin, insbesondere Humanalbumin, und Albumin-ähnlichen Polypeptiden.

## Claims

1. A sorption agent for removing protein-bound substances from a biological fluid, **characterized in that** the sorption agent comprises (a) a porous, neutral, hydrophobic polymer and (b) a polypeptide layer adsorbed on the polymer, wherein the neutral, hydrophobic polymer has a mean pore size of 30 to 40 nm, and wherein the sorption agent is made up of microparticles, the microparticles having a particle size of 20 µm or less.

2. The sorption agent according to Claim 1, **characterized in that** the neutral, hydrophobic polymer is a styrene/divinyl benzene copolymer.

3. The sorption agent according to Claim 1 or 2, **characterized in that** the polypeptide layer comprises at least one type of polypeptide, the at least one type of polypeptide being selected from albumin, in particular human albumin, and albumin-like polypeptides.

4. The sorption agent according to one of Claims 1 to 3, **characterized in that** the microparticles have a particle size of 8 µm or less.

5. The sorption agent according to Claim 4, **characterized in that** the microparticles have a particle size of 5 µm or less.

6. The sorption agent according to one of Claims 1 to 5 for treatment of reduced liver function or liver failure by bringing the sorption agent into contact with a biological fluid comprising protein-bound substances.

7. The sorption agent according to one of Claims 1 to 5 for treatment of kidney insufficiency by bringing the sorption agent into contact with a biological fluid comprising protein-bound substances.

8. The sorption agent according to Claim 6 or 7, **characterized in that** the biological fluid is a cell-free biological fluid, in particular blood plasma.

9. The sorption agent according to Claim 6 or 7, **characterized in that** the biological fluid is a biological fluid containing cells, in particular blood.

10. The sorption agent according to one of Claims 6 to 9, **characterized in that** the protein-bound substances are bilirubin, aromatic amino acids, bile acids, phenols, and mercaptans.

11. A sorption apparatus, containing a sorption agent according to one of Claims 1 to 10.

12. A plasma circuit, containing a suspension of a sorption agent according to one of Claims 1 to 10.

13. A Microsphere-based Detoxification System (MDS), containing a suspension of a sorption agent according to one of Claims 1 to 10.

14. A method for producing a sorption agent for removing protein-bound substances from a biological fluid, **characterized in that** a porous, neutral, hydrophobic polymer is adsorptively coated with a polypeptide layer, wherein the neutral, hydrophobic polymer has a mean pore size of 30 to 40 nm, and wherein the sorption agent is made up of microparticles, the microparticles having a particle size of 20 µm or less, and wherein an up to 10% polypeptide solution is used for the adsorptive coating of the neutral, hydrophobic polymer with the polypeptide layer.

15. The method according to Claim 14, **characterized in that** a 1% polypeptide solution is used for the adsorptive coating of the neutral, hydrophobic polymer.

16. The method according to Claim 14, **characterized in that** a 10% polypeptide solution is used for the adsorptive coating of the neutral, hydrophobic polymer.

17. The method according to one of Claims 14 to 16, **characterized in that** the polypeptide solution contains at least one type of polypeptide, the polypeptide being selected from albumin, in particular human albumin, and albumin-like polypeptides.

## Revendications

1. Agent de sorption pour l'élimination de substances liées à des protéines à partir d'un liquide biologique, **caractérisé par le fait que** l'agent de sorption présente (a) un polymère hydrophobe, neutre, poreux et (b) une couche de polypeptide adsorbée sur le polymère, le polymère hydrophobe, neutre, présentant une taille moyenne de pore de 30 à 40 nm et l'agent de sorption se composant de microparticules, les microparticules présentant une taille de particule de 20 *µ*m ou moins.

2. Agent de sorption selon la revendication 1, **caractérisé par le fait que** le polymère hydrophobe, neutre, est un copolymère styrène/divinylbenzène.

3. Agent de sorption selon l'une des revendications 1 ou 2, **caractérisé par le fait que** la couche de polypeptide se compose d'au moins une sorte de polypeptide, la au moins une sorte de polypeptide étant choisie parmi l'albumine, en particulier l'albumine humaine, et les polypeptides analogues à l'albumine.

4. Agent de sorption selon l'une des revendications 1 à 3, **caractérisé par le fait que** les microparticules présentent une taille de particule de 8 *µ*m ou moins.

5. Agent de sorption selon la revendication 4, **caractérisé par le fait que** les microparticules présentent une taille de particule de 5 *µ*m ou moins.

6. Agent de sorption selon l'une des revendications 1 à 5 pour le traitement de troubles de la fonction hépatique ou de l'insuffisance hépatique par mise en contact de l'agent de sorption avec un liquide biologique contenant des substances liées à des protéines.

7. Agent de sorption selon l'une des revendications 1 à 5 pour le traitement de l'insuffisance rénale par mise en contact de l'agent de sorption avec un liquide biologique contenant des substances liées à des protéines.

8. Agent de sorption selon l'une des revendications 6 ou 7, **caractérisé par le fait que** le liquide biologique est un liquide biologique exempt de cellules, en particulier le plasma sanguin.

9. Agent de sorption selon l'une des revendications 6 ou 7, **caractérisé par le fait que** le liquide biologique est un liquide biologique contenant des cellules, en particulier le sang.

10. Agent de sorption selon l'une des revendications 6 à 9, **caractérisé par le fait qu**'il s'agit, dans le cas des substances liées à des protéines, de la bilirubine, d'acides aminés aromatiques, d'acides biliaires, de phénols et de mercaptans.

11. Dispositif de sorption, contenant un agent de sorption selon l'une des revendications 1 à 10.

12. Circuit de plasma, contenant une suspension d'un agent de sorption selon l'une des revendications 1 à 10.

13. Système de désintoxication à base de microsphères (MSD), contenant un agent de sorption selon l'une des revendications 1 à 10.

14. Procédé de fabrication d'un agent de sorption pour l'élimination de substances liées à des protéines à partir d'un liquide biologique, **caractérisé par le fait qu**'un polymère hydrophobe, neutre, poreux, est revêtu par adsorption par une couche de polypeptide, le polymère hydrophobe, neutre, présentant une taille moyenne de pore de 30 à 40 nm et l'agent de sorption se composant de microparticules, les microparticules présentant une taille de particule de 20 *µ*m ou moins, et une solution de polypeptide jusqu'à 10% étant utilisée pour le revêtement adsorptif du polymère hydrophobe, neutre, par la couche de polypeptide.

15. Procédé selon la revendication 14, **caractérisé par le fait qu**'une solution de polypeptide à 1% est utilisée pour le revêtement adsorptif du polymère hydrophobe, neutre.

16. Procédé selon la revendication 14, **caractérisé par le fait qu**'une solution de polypeptide à 10% est utilisée pour le revêtement adsorptif du polymère hydrophobe, neutre.

17. Procédé selon l'une des revendications 14 à 16, **caractérisé par le fait que** la solution de polypeptide contient au moins une sorte de polypeptide, le polypeptide étant choisi parmi l'albumine, en particulier l'albumine humaine, et les polypeptides analogues à l'albumine.
